(19) Europäisches Patentamt
European Patent Office
Office européen des brevets

(11) Publication number: 0 118 750 B1

(12) EUROPEAN PATENT SPECIFICATION

(45) Date of publication of patent specification: 04.04.90

(51) Int. Cl.[5]: C 12 P 1/00, C 12 P 19/36 // C12N9/04, C12P7/26, C12P17/02, C12P19/02

(21) Application number: 84101220.6

(22) Date of filing: 07.02.84

# (54) Regeneration of NAD(P) cofactor.

(30) Priority: 09.02.83 US 465080

(43) Date of publication of application: 19.09.84 Bulletin 84/38

(45) Publication of the grant of the patent: 04.04.90 Bulletin 90/14

(84) Designated Contracting States: CH DE FR GB IT LI SE

(56) References cited:
FR-A-2 217 296

ANALYTICAL LETTERS, vol. 9, no. 3, 1976, pages 257-276; W.H.BARICOS et al.: "Practical processing with cofactor-requiring enzymes

(73) Proprietor: PHILLIPS PETROLEUM COMPANY
5th and Keeler
Bartlesville Oklahoma 74004 (US)

(72) Inventor: Hopkins, Thomas R.
3417 S.E. Willowood Dr.
Bartlesville Oklahoma 74006 (US)

(74) Representative: Dost, Wolfgang, Dr.rer.nat., Dipl.-Chem. et al
Patent- und Rechtsanwälte Bardehle-Pagenberg-Dost-Altenburg & Partner Postfach 86 06 20
D-8000 München 86 (DE)

The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to the regeneration of the cofactor NAD(P) using a multi-enzyme system. More specifically, this invention relates to an improved, more efficient process for the regeneration of NAD(P) which is required as a coenzyme or cofactor in oxidative reactions catalyzed by dehydrogenase enzymes.

There are over 2500 known enzymes and over one-third of these require cofactor molecules to function as catalysts. The enzymes which require nicotine-adenine-dinucleotide, more commonly called NAD or nicotine-adenine-dinucleotide-phosphate, NADP, are called dehydrogenases or reductases. Collectively, these two cofactors are referred to as NAD(P). These enzymes which require cofactors are being increasingly used to achieve selective chemical conversions. For example, U.S. 3,331,752 discloses that NAD(P) is used as a cofactor to aid dehydrogenases in oxidizing substrates while simultaneously the NAD(P) is transformed to its reduced form NAD(P)$H_2$.

It is also known in the art that NAD(P) can be regenerated from NAD(P)$H_2$. U.S. 3,862,012 discloses that NAD(P) is regenerated from NAD(P)$H_2$ in the presence of acetaldehyde and alcohol dehydrogenase.

The combination of reactions into one system wherein NAD(P) is reduced to NAD(P)$H_2$ and subsequently regenerated therefrom has been disclosed in the art as, for example, in *Enzyme Engineering*, vol. 3, pp. 371—377 (1978). This system may be generalized by the following reactions:

$$\text{(1)}\quad \text{reduced substrate} + \text{NAD(P)} \xrightarrow[\text{dehydrogenase}]{\text{substrate}} \text{oxidized substrate} + \text{NAD(P)H}_2.$$

$$\text{(2)}\quad \text{NAD(P)H}_2 + \text{aldehyde} \xrightarrow[\text{dehydrogenase}]{\text{alcohol}} \text{alcohol} + \text{NAD(P)}$$

The overall reaction, therefore, is:

$$\text{(3)}\quad \text{reduced substrate} + \text{aldehyde} \longrightarrow \text{oxidized substrate} + \text{alcohol}$$

Because of the relative high cost of NAD(P), its efficient regeneration is highly desirable. While the above system accomplishes the latter, it still has some disadvantages. For example, the formation of stoichiometric amounts of alcohol from aldehyde as shown in equation (3) complicates the isolation and purification of the oxidized substrate. If the alcohol was not present, then isolation and purification of the oxidized substrate would be more readily accomplished.

A further disadvantage of the above two enzyme system lies in the fact that many NAD(P) cofactor-linked dehydrogenase reactions are readily reversible and their energy driving force, commonly called $\Delta G$, can be relatively small. Therefore, large concentrations of aldehyde must often be maintained in a two enzyme reaction mixture in order to drive reaction (3) toward completion. There is a practical limit to the amount of aldehyde which a reaction mixture can contain before the solvent properties are altered to such an extent to inhibit the enzyme catalysts.

It would be highly desirable to have a system whereby NAD(P) is efficiently regenerated yet overcomes the above-mentioned difficulties.

Therefore, an object of this invention is to provide an improved process wherein NAD(P) is efficiently regenerated from NAD(P)$H_2$ without having to have present large amounts of a cosubstrate such as an aldehyde and without encountering purification problems of the oxidized substrate due to the presence of reaction products such as alcohols from the corresponding cosubstrate.

Other aspects, objects, and advantages of the present invention will become apparent from the specification and the claims.

In accordance with the present invention, I have discovered that in a process wherein a first substrate is oxidized and NAD(P) is reduced in the presence of a first NAD(P) cofactor-linked oxidoreductase enzyme to form an oxidized first reaction product and NAD(P)$H_2$; and subsequently, a second substrate and NAD(P)$H_2$ are reacted in the presence of a second NAD(P) cofactor-linked oxidoreductase enzyme to form a second reaction product and NAD(P), an improvement is obtained which comprises reacting the resulting second reaction product with oxygen in the presence of an oxygen-linked oxidoreductase enzyme to regenerate the corresponding aldehyde.

My invention can be best understood from the following system of equations:

$$\text{(4)}\quad \text{Substrate}_1 + \text{NAD(P)} \xrightarrow{E_1} \text{Reaction Product}_1 + \text{NAD(P)H}_2$$

$$\text{(5)}\quad \text{Substrate}_2 + \text{NAD(P)H}_2 \xrightarrow{E_2} \text{Reaction Product}_2 + \text{NAD(P)}$$

$$(6) \quad \text{Reaction Product}_2 + O_2 \xrightarrow{E_3} \text{Substrate}_2 + H_2O_2$$

In this case the overall reaction will be:

$$(7) \quad \text{Substrate}_1 + O_2 \longrightarrow \text{Reaction Product}_1 + H_2O_2$$

Equations (4) and (5) illustrate processes which are known in the art. $E_1$ and $E_2$ both represent NAD(P) cofactor-linked oxidoreductase enzymes. My invention consists of the addition of step (6) to the overall process. By reacting the Reaction Product$_2$ ($P_2$) formed in Equation (5) with oxygen, catalyzed by $E_3$, an oxygen-linked oxidoreductase enzyme, the continual recycle of ($P_2$) is achieved as well as the continual regeneration of Substrate$_2$ ($S_2$). Only trace amounts of ($S_2$) are needed in the net reaction and ($P_2$) does not accumulate thereby complicating the purification of the Reaction Product$_1$ ($P_1$). In addition, the equilibrium of reaction (6) is essentially all the way to the right. Thus the 3 enzyme reaction (equations 4, 5, and 6) is also driven to completion. This makes the reaction very efficient and also simplifies product isolation. If desired, the $H_2O_2$ may be removed, if desired, by the addition of catalase to the reaction mixture.

In the practice of the present invention any cofactor-linked oxidoreductase enzyme as illustrated in equation (4) may be utilized. A cofactor-linked oxidoreductase enzyme may be defined as any enzyme which simultaneously reduces NAD(P) to NAD(P)$H_2$ and oxidizes Substrate$_1$ ($S_1$). This includes the broad class of enzymes called dehydrogenases and reductases which use NAD(P). According to the system of the International Enzyme Commission these enzymes would be placed into the following category numbers: 1.1.1; 1.2.1; 1.3.1; 1.4.1; 1.5.1; 1.6.1; and 1.8.1. For a detailed outline of the numbering system see M. Dixon and E. C. Webb, *Enzymes*, 2nd Ed., (Academic Press, N.Y. 1964), pp. 210—215. Examples of such enzymes are dehydrogenases such as alcohol dehydrogenase, glycerolphosphate dehydrogenase, histidinol dehydrogenase, shikimate dehydrogenase, lactate dehydrogenase, 3-hydroxyaryl-CoA dehydrogenase, malate dehydrogenase, isocitrate dehydrogenase, glucose-6-phosphate dehydrogenase, formate dehydrogenase, horse liver alcohol dehydrogenase, glucose dehydrogenase, amino acid dehydrogenase, sorbitol dehydrogenase, 20-β-hydroxysteroid dehydrogenase and formaldehyde dehydrogenase.

Typical $S_1$'s include alcohols, steroids, esters, amino acids, nitrates, and sugars. Specific examples of such substrates are cyclohexanol, 4,5-dihydro-uracil, 5,10-methylenetetrahydrofolate, β-D-glucose and L-glutamate. Whatever first substrate is used, it must be oxidized by $E_1$, the first NAD(P) cofactor-linked oxidoreductase enzyme.

The enzyme utilized to regenerate NAD(P) and form $P_2$ as in Equation (5) should be another NAD(P) cofactor-linked oxidoreductase enzyme which will act on the second substrate, $S_2$. Examples of such second cofactor-linked oxidoreductase enzymes which can be utilized include alcohol dehydrogenase, glucose dehydrogenase, xanthine dehydrogenase, malate dehydrogenase, lactate dehydrogenase, aldehyde dehydrogenase, oxalate dehydrogenase, glycerol dehydrogenase and sulfate reductase.

If the enzyme has a broad specification, $E_1$ and $E_2$ can be the same enzyme. A specific example is horse liver alcohol dehydrogenase which catalyzes hundreds of different reactions in the enzyme category EC 1.1.1.

Typically, $S_2$ is an aldehyde such as acetaldehyde and $P_2$ will be the corresponding alcohol such as ethanol or $S_2$ is a ketone such as cyclohexanone and $P_2$ is again the corresponding alcohol such as cyclohexanol.

An oxidase enzyme is employed to convert $P_2$ back to $S_2$. An oxidase enzyme may be defined as one which oxidizes its substrate by removing two hydrogen atoms from the substrate and transferring them to oxygen to generate $H_2O_2$. Examples of such enzymes include alcohol oxidase, glucose oxidase, xanthine oxidase, malate oxidase, lactate oxidase, aldehyde oxidase, glycerol oxidase, and sulfite oxidase.

In the present invention, $E_2$ and $E_3$ must both be selected such that they operate in compatible systems. This means that for whatever $E_2$ is used to convert $S_2$ to $P_2$, $E_3$ must be chosen such that $P_2$ is oxidized back to $S_2$. For example, should $E_2$ be malate dehydrogenase in Equation (5), $E_3$ would be the corresponding enzyme malate oxidase in Equation (6). Other exemplary combinations of $E_2/E_3$ for use in the present invention are alcohol dehydrogenase/alcohol oxidase; horse liver alcohol dehydrogenase/alcohol oxidase; aldehyde dehydrogenase/aldehyde oxidase; glycerol dehydrogenase/glycerol oxidase; sulfate reductase/sulfite oxidase; and lactate dehydrogenase/lactate oxidase.

Therefore, in the present invention, the overall reaction, Equation (7) will remain unchanged. The reaction will only require catalytic amounts of NAD(P) or NAD(P)$H_2$ for the reaction given by Equations (4) and (5) and catalytic amounts of either the substrates or products common to a given $E_2/E_3$ combination for the reactions given by Equations (5) & (6).

The above-cited enzymes are all known in the art and many are available from commercial supply houses such as Sigma Chemical Company, St. Louis, Missouri.

Alcohol oxidase enzymes are known in the art to be produced by various methanol-utilizing microorganisms. A particularly suitable alcohol oxidase is that derived from the microorganism *Pichia pastoris* by the following procedure.

An aqueous suspension of cells of *Pichia pastoris* microorganism is prepared. The aqueous fluid containing a suspension of cells is homogenized to produce a homogenate. Suspended solids are removed

from the homogenate to produce a crude solution containing soluble alcohol oxidase. The crystalline form of the enzyme can be obtained by methods of ultra filtration, presently preferably and conveniently by dialysis, or by other separation methods. Dialysis involves dialyzing the crude solution, prepared by homogenizing an aqueous fluid having a cell density effective for crystallization of alcohol oxidase in a recovery range solution having a molar ionic strength in the range of between 0.05 *M* and about 0.01 *M*, against a dialysis medium across a membrane impermeable to the alcohol oxidase but permeable to dialysis medium water and buffer molecules, if any, to achieve on the enzyme side of the membrane the recovery range solution thereby resulting in crystalline alcohol oxidase, and separating the resulting crystalline alcohol oxidase from the dialysis medium. Such an enzyme isolation procedure is shown in Example I.

The enzyme is characterized by the following properties. It has 8 subunits of an estimated molecular weight of 78,000 per subunit as determined by SDS gel electrophoresis. The enzyme is a flavoprotein having FAD (flavin adenine dinucleotide) coenzyme. The apparent Michaelis constant Km for methanol is about 0.7. The enzyme is further characterized by its reacting with various short chain primary alcohols.

Alcohol dehydrogenase enzymes are known in the art. They are commonly isolated from yeast, bacteria and animal sources. Dehydrogenase enzymes obtained from yeast and animal sources are specific for the cofactor NAD. Dehydrogenase enzymes isolated from bacterial sources such as *Leuconostoc* are specific for the cofactor NADP.

In the process of the present invention, the reaction ingredients are combined in amounts which must be optimized for each particular combination of enzyme selected for Equations (4), (5) and (6). For example, one must consider the substrate concentration, the stability and physical form of the enzyme (soluble versus immobilized), and the nature of the solvent (pH, presence of solvents, ionic strength, etc.) in Equation (4). These conditions will effect the performance of the recycle system in Equations (5) and (6). The following combination of ingredients will serve as a starting approximation: Equation (4); 1—10% (w/v) substrate in aqueous solution buffered for the pH optimum of the selected dehydrogenase enzyme. The enzyme is present at a concentration of 0.1—1 enzyme units/mL media. Equations (5) and (6); yeast alcohol dehydrogenase and yeast alcohol oxidase at a concentration of 0.1—3 enzyme units/mL media and final concentration of NAD of $10^{-4}$—$10^{-6}$ *M* and acetaldehyde or alcohol at $3 \times 10^{-2}$ *M*. The solution is incubated at 20 to 40°C with gentle stirring in an open vessel in order that oxygen in the air can freely diffuse into the reaction.

In another embodiment, some substrates are added gradually to the reaction mixture. This is because the presence of large amounts of substrate may inhibit the enzyme system.

The same or all of the enzymes in the reaction system could be immobilized in a reactor to aid in subsequent recovery or added stabilization of the enzymes. It is also possible to immobilize the nicotinamide coenzymes for the same reasons.

The following examples further illustrate the present invention.

## Example I

In a continuous aerobic fermentation process, methanol and an aqueous mineral salts medium in a volume ratio of about 40 to 60, respectively, were fed individually to a fermenter, inoculated with the yeast species *Pichia pastoris* NRRL Y—11430, at a rate so that methanol is the growth-limiting factor. The fermenter was a 1500-liter foam-filled fermenter with a liquid volume of about 610 liters, with automatic pH, temperature, and level control. Agitation was provided by two conventional paddle-type turbines driven at 1000 rpm. The aeration rate was about 4 volumes of air (at about 38 psig and about 25°C) per volume of ferment in the fermenter per minute. Anhydrous ammonia was added at such a rate as to maintain the pH of the fermentation mixture at about 3.5.

The aqueous mineral salts medium was prepared by mixing, with each liter of tap water, 15.86 mL 75 percent $H_3PO_4$, 9.53 g $K_2SO_4$, 7.8 g $MgSO_4 \cdot 7H_2O$, 0.6 g $CaCl_2 \cdot 2H_2O$, and 2.6 g 85 percent KOH. The trace mineral solution plus biotin was fed separately via the methanol stream at a rate of 10 mL per liter of methanol. The trace mineral solution plus biotin was prepared by mixing 780 mL of a trace mineral solution, 20 mL water, 200 mL methanol and 0.032 g biotin.

The trace mineral solution was prepared by mixing, for each liter of solution, 65 g $FeSO_4 \cdot 7H_2O$, 20 g $ZnSO_4 \cdot 7H_2O$, 3.0 g $MnSO_4 \cdot H_2O$, 6.0 g $CuSO_4 \cdot 5H_2O$, 5.0 mL conc. $H_2SO_4$, and sufficient deionized water to make 1 liter of solution.

The aqueous mineral salts medium was fed at a rate of 31.5 liters per hour and the methanol at a rate of 21 liters per hour.

The fermentation was conducted at about 30°C and about 38 psig pressure, with a fermentation time of 11.6 hours.

For analytical purposes, the resulting yeast cells were separated from the fermentation effluent (ferment) by centrifugation, washed by suspension in water and recentrifugation, dried overnight at 100°C, and weighed. On a dried basis, the yield of yeast cells typically was about 40.6 g per 100 g of methanol fed. The cell density typically was about 128.4 g of cells per liter of fermenter effluent. The total solids content of the ferment typically was about 134.7 g per liter, cells plus dissolved solids. A portion of the fermenter effluent was frozen and stored.

Fermentation of *Pichia pastoris* NRRL Y—11430 was carried out by a method of which that set forth

above is typical. A portion of the fermenter effluent was removed and adjusted to pH 7.5 with ammonium hydroxide, and was homogenized on a Dyno-Mill® Model KDL using a 0.6 liter vessel in a continuous operation at 30°C using belt combination #3 and a flow of 20—30 mL/hr. The beads in the mill were lead-free glass beads with a diameter of 0.3—0.5 mm. The resulting homogenate was centrifuged at 5°C and 20,000 × g for 30 minutes to yield a cell-free supernatant. The cell-free supernatant enzyme activity measured by the dye-peroxidase method described below was about 330 EU/mL. The supernatant was stored frozen for future use. This describes the preparation of crude yeast homogenate.

Six 130 mL portions of the supernatant were placed in cellulose acetate dialysis bags and dialyzed at 5°C against about 8 liters of distilled water. After 4 days, the aqueous phase of each bag was decanted. The solids remaining in the bags consisted of two types of solid. The thin upper white layer was carefully removed and discarded. The bottom solid was brown-yellow and was crystalline alcohol oxidase. A portion of the crystalline alcohol oxidase was dissolved in distilled water (about 10 times the volume of the solid) and an assay by the dye-peroxidase method described below showed an activity of 94 EU/mL. The specific activity of the alcohol oxidase was 10.4 EU/mg of protein.

A sample of the solid alcohol oxidase was examined by SDS gel electrophoresis and a single band was observed indicating a homogeneously pure enzyme. A comparison of electrophoretic mobility with those of proteins having known molecular weight indicates a subunit molecular weight of about 72,000 ± 3000 (estimated). This describes the preparation of pure yeast alcohol oxidase from *Pichia pastoris*.

The alcohol oxidase activity for reaction with methanol was determined by the dye-peroxidase method. A dye-buffer mixture was prepared by mixing 0.1 mL of an o-dianisidine solution (1 weight percent o-dianisidine in water) with 12 mL of aerated 0.1 M sodium phosphate buffer (pH 7.5). The assay mixture was prepared with 2.5 mL of the dye-buffer mixture, 50 µL of methanol, 10 µL of a peroxidase solution (1 mg of horse-radish peroxidase-Sigma, Type II), and 25 µL of the alcohol oxidase solution. The assay mixture was maintained at 25°C in a 4 × 1 × 1 cm cuvette and the increase in absorbance by the dye at 460 nm was recorded for 2 to 4 minutes. The enzyme activity was calculated by

$$\text{Activity } (\mu \text{ mole/min/mL or Enzyme Units/mL}) = \frac{\Delta A}{\text{min.}} \times 11.5$$

wherein 11.5 is a factor based on a standard curve prepared with known aliquots of $H_2O_2$ and $\Delta A$ is the change in absorbance during the experimental interval.

Example II

Two control experiments were carried out where one of the enzymes required for the inventive cofactor regeneration was omitted. The following were placed in a test tube and mixed:

0.2 mL of 10 m*M* cyclohexanol in 0.05 *M* phosphate buffer (pH 8)
0.01 mL of 2.5 m*M* NAD (1 mg/mL)
0.1 mL of 100 m*M* acetaldehyde

Concentrated alcohol oxidase (AO) purified as described above (0.01 mL of 10 mg/mL solution) was added to control #1, and 0.01 mL of horse liver alcohol dehydrogenase (HL—ADH), (1 mg/mL solution) was added to control #2. A third sample (designated "standard") was mixed containing both AO and ADH as indicated above. These were assayed by GC after the indicated reaction time had elapsed:

| | | GC Area % | |
|---|---|---|---|
| Sample | Reaction time | Cyclohexanol | Cyclohexanone |
| Control #1 | 1 hr 45 min | 100 | — |
| Control #2 | 1 hr | 70.6 | 29.4 |
| Standard | 1 hr 20 min | 38.6 | 61.4 |

This example indicates that cyclohexanol undergoes essentially no reaction in the absence of horse liver alcohol dehydrogenase (control #1). In the presence of alcohol dehydrogenase but the absence of alcohol oxidase, some conversion of cyclohexanol is observed after one hour (control #2). This is expected since alcohols are known to undergo reaction with oxidized cofactor, NAD(P) in the presence of horse liver ADH to give ketones:

$$\text{Cyclohexanol} \underset{\text{NAD(P)H}_2}{\overset{\text{NAD(P)}}{\rightleftharpoons}} \text{Cyclohexanone} \qquad (1)$$

An additional reaction promoted by ADH is:

$$\text{Acetaldehyde} \underset{\text{NAD(P)}}{\overset{\text{NAD(P)H}_2}{\rightleftharpoons}} \text{Ethanol} \qquad (2)$$

Taken together, reactions (1) and (2) are exemplary of a classical two-substrate cofactor regeneration scheme. In this example, the conversion of cyclohexanol to cyclohexanone according to equation (1) above generates reduced cofactor, $NAD(P)H_2$. Reduced cofactor in the presence of acetaldehyde and HL—ADH generates ethanol and regenerates oxidized cofactor NAD, as shown in equation (2) above. This regenerated NAD can promote conversion of additional cyclohexanol to cyclohexanone until the concentrations of primary reaction product (cyclohexanone) and secondary reaction product (ethanol) reacts such a level that the overall coupled reaction comes to equilibrium, at which point the reaction stops.

The standard run above shows that in the presence of alcohol oxidase and alcohol dehydrogenase, reaction occurs considerably faster. Here, a third enzymatic conversion is taking place, this one in the presence of alcohol oxidase:

$$\text{Ethanol} \xrightarrow{O_2} \text{Acetaldehyde} \qquad (3)$$

This reaction is essentially irreversible so that the steady state concentration of ethanol is virtually zero while a steady state concentration of acetaldehyde is maintained. By maintaining effective concentrations of acetaldehyde (via equation 3), reduced cofactor can be continuously converted back to oxidized form as acetaldehyde is again reduced to ethanol (via equation 2), which in turn continuously provides oxidized cofactor necessary for the desired conversion illustrated by equation 1. Thus, the essentially irreversible nature of equation 3 allows one to drive an equilibrium reaction such as that illustrated by equation 1 in one direction and thus in time to completion.

## Example III

The following reagents were charged to a test tube, mixed and sampled for GC analysis:

0.2 mL of 10 m*M* cyclohexanol in 0.05 *M* phosphate buffer (pH 8)
0.01 mL of 2.5 m*M* NAD (1 mg/mL)
0.01 mL of horse liver ADH (1 mg/mL)
0.1 mL of 100 m*M* acetaldehyde

After 15 minutes, 0.01 mL of concentrated (~10 mg/mL) alcohol oxidase was added. Test tube contents were sampled intermittently over several hours for analysis by GC.

| Sample time | GC Area % Cyclohexanol | Cyclohexanone |
|---|---|---|
| 0 | 81.6 | 18.4 |
| 10 min. | 60.2 | 39.8 |
| 35 min. | 42.1 | 57.9 |
| 1 hr. | 28.3 | 71.7 |
| 1 hr. 25 min. | 19.3 | 80.7 |
| 1 hr. 50 min. | 12.2 | 87.8 |
| 23 hr. 30 min. | — | 100 |

A graph of GC peak area vs. hours of reaction gives a smooth, non-linear curve which indicates that reaction is complete in 3—4 hours with a $t_{\frac{1}{2}}$ of about 45 minutes. Yield based on m*M* cyclohexanol charged is about 78%, which indicates an NAD turnover of about 62.

This example demonstrates that the combination of alcohol oxidase, alcohol dehydrogenase and acetaldehyde cause regeneration of cofactor NAD in the presence of a reactive substrate such as cyclohexanol and drive the primary reaction (cyclohexanol to cyclohexanone) to completion.

## Example IV

A series of solutions were prepared with varying amounts of cyclohexanol, NAD, horse liver ADH, AO and acetaldehyde in a test tube. Total solution volume was brought to 0.33 mL with aerated potassium

phosphate buffer (0.05 *M*; pH 8). All samples were incubated for 1 hour, then assayed by GC for cyclohexanol disappearance and cyclohexanone formation. The standard reagent mix was as follows:

0.2 mL of 10 m*M* cyclohexanol
0.01 mL of 2.5 m*M* NAD
0.001 mL of HL—ADH (10 mg/mL)
0.01 mL of AO (10 mg/mL)
0.01 mL of 1 *M* acetaldehyde

In each of the following experiments, the concentration of one reagent at a time was varied from that specified above:

| Solution Variant | GC Area % Cyclohexanol | GC Area % Cyclohexanone |
|---|---|---|
| 0.1 mL (10 m*M*) cyclohexanol | 0 | 100 |
| None | 39.3 | 60.7 |
| None | 38.7 | 61.3 |
| 0.05 mL (100 m*M*) cyclohexanol | 65.8 | 34.2 |
| 0.1 mL (100 m*M*) cyclohexanol | 80.6 | 19.4 |
| 0.2 mL (100 m*M*) cyclohexanol | 90.2 | 9.8 |
| 0.1 mL NAD | 47.1 | 52.9 |
| 0.01 mL HL—ADH | 24.3 | 75.7 |
| 0.001 mL AO | 56.6 | 43.4 |
| 0.001 mL acetaldehyde | 83.3 | 16.7 |
| 0.05 mL acetaldehyde | 15.0 | 85.0 |

It can be seen from inspection of these data that best results are obtained with less than 0.01 mL of 2.5 m*M* NAD, about 0.01 mL each of HL—ADH and AO (both 10 mg/mL) and greater than 0.001 mL of 1 *M* acetaldehyde.

## Example V

Additional studies were carried out to determine the effect of reagent variation in the inventive cofactor regeneration scheme. The standard reagent mix was as follows:

0.1 mL of 100 m*M* cyclohexanol
0.01 mL of 2.5 m*M* NAD
0.01 mL of HL—ADH (1 mg/mL)
0.005 mL of AO (10 mg/mL)
0.01 mL of 1 *M* acetaldehyde

As in Example V, one reagent at a time was varied, reaction was carried out for 1 hour in a total volume of 0.3 mL (0.05 *M* aerated potassium phosphate buffer, pH 8.0 added as needed).

| Solution Variant | GC Area % Cyclohexanol | GC Area % Cyclohexanone |
|---|---|---|
| a) Cyclohexanol (100 m*M*): | | |
| 0.01 mL | 38.7 | 61.3 |
| 0.05 mL | 82.9 | 17.1 |
| 0.1 mL | 84.7 | 15.3 |
| 0.2 mL | 90.9 | 9.1 |
| b) NAD (2.5 m*M*): | | |
| 0.001 mL | 87.5 | 12.5 |
| 0.005 mL | 84.9 | 15.1 |
| c) HL—ADH (1 mg/mL) | | |
| 0.001 mL | 91.4 | 8.6 |
| 0.005 mL | 84.7 | 15.3 |
| 0 | 96.4 | 3.6 |
| d) AO (10 mg/mL) | | |
| 0 | 91.7 | 8.3 |

These results indicate that conversion of cyclohexanol is strongly dependent on the concentration of alcohol dehydrogenase. In addition, the reaction is relatively insensitive to the concentration of oxidized cofactor, NAD, since the cofactor is continuously being replenished by the inventive regeneration scheme.

Example VI

Additional studies were carried out to determine the effect of long sample incubation periods (18—24 hrs.) as well as reagent concentration variation in the inventive cofactor regeneration scheme. The standard reagent mix used was as follows:

0.1 mL of 100 m*M* cyclohexanol
0.005 mL of 2.5 m*M* NAD
0.01 mL of HL—ADH (1 mg/mL)
0.01 mL of AO (10 mg/mL)
0.01 mL of 1 *M* acetaldehyde

As above, one or more reagents at a time were varied from the standard values indicated. All solutions were brought to a total of 0.3 mL with aerated potassium phosphate buffer (0.05 *M*; pH 8.5).

| Entry # | Solution Variant | GC Area % Cyclohexanol | GC Area % Cyclohexanone |
|---|---|---|---|
| 1 | None | 49.7 | 50.3 |
| 2 | 0.01 mL cyclohexanol | 22.4 | 77.6 |
| 3 | 0.01 mL cyclohexanol & 0.001 mL NAD | 21.6 | 78.4 |
| 4 | 0.001 mL NAD | 52.3 | 47.7 |
| 5 | 0 mL NAD | 82.9 | 17.1 |
| 6 | 0.001 mL ADH | 79.2 | 20.8 |
| 7 | 0 mL ADH | 88.1 | 11.9 |
| 8 | 0.001 mL AO | 39.1 | 60.9 |
| 9 | 0 mL AO | 92.9 | 7.1 |
| 10 | 0.001 mL acetaldehyde | 84.6 | 15.4 |
| 11 | 0 mL acetaldehyde | 84.2 | 15.8 |

These results indicate that for long incubation periods, the inventive cofactor regeneration scheme is relatively insensitive to NAD concentration (compare entries 1 vs. 4 and 2 vs. 3, corresponding to a 10-fold variation) and AO concentration (compare entries 1 and 8). In addition, it is demonstrated that reaction proceeds poorly in the absence of NAD, ADH, AO, or acetaldehyde (See entries 5, 7, 9, and 11.). It is noted that even in the absence of acetaldehyde some primary product (cyclohexanone) is produced from cyclohexanol (see entry 11). The likely explanation for this is the observation that some of our reagents (NAD and ADH) are contaminated with traces of ethanol. These catalytic amounts are enough to support the recycle scheme which is the subject of this application. Further support for this interpretation is found with entries 9 and 10 where it is seen that in the complete system traces of acetaldehyde (and by *infra*, ethanol) will support the recycle scheme.

Example VII

The following reagents were admixed in a small beaker:

2.7 mL aerated potassium phosphate buffer (0.05 *M*; pH 8)
0.01 mL of NAD solution (10 mg/mL or 0.025 *M*)
0.5 mL horse liver-alcohol dehydrogenase solution (Sigma-1 mg/mL)
0.1 mL of 1 *M* acetaldehyde
0.1 mL of saturated alcohol oxidase (~10 mg/mL, prepared as described in example I)

A dissolved oxygen probe, connected to an electronic signal differentiator, was placed in the above prepared solution. The relative decrease in dissolved oxygen level (ADO) was monitored upon addition of 0.1 mL of 2.5 vol% solutions of several alcohols:

| Added Alcohol | ΔDO |
|---|---|
| 4-penten-1-ol | * |
| 9-decen-1-ol | Yes |
| 3-buten-1,4-diol | Yes |
| 3-buten-2-ol | Yes |
| 2-bromoethanol | * |
| glycidol | * |
| 2-butanol | Yes |
| isobutyl alcohol | * |
| isopropyl alcohol | * |
| tertiary amyl alcohol | No |
| tertiary butanol | No |
| 1,4-butanediol | Yes |
| cyclohexanol | Yes |

* Methanol contamination of reagent chemicals did not allow assay to be completed.

This example illustrates that the inventive cofactor regeneration scheme will operate with horse liver alcohol dehydrogenase on a variety of alcohol substrates.

Example VIII

The following reagents were placed in a 4 mL cuvette:

2 mL 0.2 *M* sorbitol
0.1 mL sorbitol dehydrogenase (0.5 mg/mL)
0.1 mL NAD (0.01 *M*)
0.1 mL yeast alcohol dehydrogenase (2 mg/mL)
0.1 mL acetaldehyde (0.01 *M*)

All reagents were dissolved in 0.05 *M* aerated potassium phosphate buffer (pH 6.5). Optical density was monitored at 340 nm which is a measure of the concentration of NADH. NAD does not absorb light at 340 nm. After about 10 minutes, the optical density began to increase, indicating the conversion of NAD to

$NADH_2$. After an additional 5—7 minutes had elapsed, 0.01 mL of concentrated (10 mg/mL) alcohol oxidase solution were added to the cuvette. There was an initial perturbation in optical density due to the added AO absorbance, and within a minute or so, the optical density dropped to a nominal level and remained unchanged for about 12 minutes when it suddenly began to increase and reached a new steady level after about 10 minutes. At this point, the cuvette was opened and agitated to reaerate the solution, at which time the optical density rapidly returned to baseline value indicating NAD was predominantly in oxidized form. After several minutes (depending on how well the cuvetted had been aerated), the optical density again suddenly increased until the sample was re-aerated again.

This example illustrates that the conversion of sorbitol to fructose can be carried out with cofactor regeneration in the presence of alcohol oxidase, alcohol dehydrogenase, acetaldehyde (or ethanol), and oxygen as shown in the following cyclic reaction scheme:

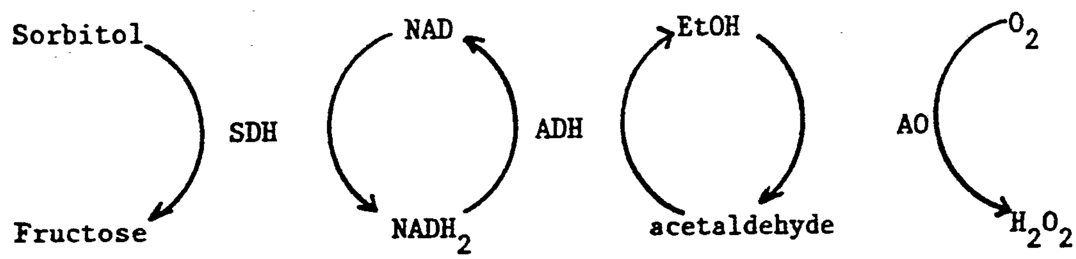


The conversion of one molecule of sorbitol to fructose transforms a molecule of NAD to a molecule of $NADH_2$. In the presence of catalytic amounts of acetaldehyde (or ethanol) and alcohol dehydrogenase, $NADH_2$ is oxidized to NAD, and ethanol is produced. The ethanol is converted in the presence of alcohol oxidase and oxygen back into acetaldehyde — thus allowing continuous regeneration of NAD cofactor. Accumulation of reduced cofactor, $NADH_2$, is only observed when insufficient oxygen is provided to maintain the sequence as outlined above.

Example IX

An open vessel equipped with a magnetic stirring bar was charged with 10 µL butane-1,4-diol, 4.2 mg NAD, 0.2 mg HL—ADH, 56 mg flavin mononucleotide (FMN) and 1 mL of 0.05 *M* glycine buffer (pH 9.0). This solution was incubated at room temperature for 24 hours. When assayed by gas-liquid chromatography (GLC) with caprolactone as internal standard on a 1/8 inch × 6 foot K20M (6% on Teflon 35/60) column at 135°C isothermal, very little conversion (<10%) to γ-butyrolactone was indicated.

A similar reaction charge was prepared using the same quantities of butene-1,4-diol, NAD, and ADH. Instead of FMN, 100 µL of concentrated AO (10 mg/mL) and 250 µL of 0.1 *M* acetaldehyde were employed, and instead of glycine buffer, 0.05 *M* sodium phosphate buffer (pH 8.0) was employed. Room temperature incubation for 24 hours resulted in nearly quantitative conversion (>90%) of butane-1,4-diol to γ-butyrolactone.

The results of these experiments demonstrate that the inventive cofactor regeneration scheme is effective for driving the equilibrium of enzymatic conversions to the right and is superior to the non-enzymatic recycle method.

Example X

Two open vessels equipped with magnetic stirring bars were charged with 10 µL of butane-1,4-diol or hexane-1,6-diol in 0.65 mL of 0.05 *M* standard phosphate buffer (pH 8) containing 250 µL of 0.1 *M* acetaldehyde, 1.0 mg NAD, 100 µL of concentrate AO (10 mg/mL) and 0.2 mg HL—ADH. These vessels were stirred at room temperature for 4 hours, then assayed for lactone formation under GLC conditions described above. A 49% yield of γ-butyrolactone and 54% yield of ε-caprolactone was obtained under these conditions.

These experiments demonstrate the operability of the inventive cofactor regeneration scheme for the production of lactone products.

Reasonable modifications and variations which will become apparent to those skilled in the art can be made in this invention without departing from the scope and spirit thereof.

## Claims

1. A process for the enzymatic oxidation of a first substrate to form a first reaction product which comprises:

(1) reacting a first substrate with a first NAD(P) cofactor-linked oxidoreductase enzyme and NAD(P) to form a first reaction product and $NAD(P)H_2$; wherein said first substrate is oxidized by said first NAD(P) cofactor-linked oxidoreductase enzyme, and

(2) subsequently reacting a second substrate and the $NAD(P)H_2$ produced in step (1) with a second NAD(P) cofactor-linked oxidoreductase enzyme to form a second reaction product and NAD(P); wherein said second substrate is reduced by said second NAD(P) cofactor-linked oxidoreductase enzyme,

characterized by

(3) reacting said second reaction product with oxygen catalyzed by an oxidase enzyme, to thereby regenerate said second substrate.

2. The process of claim 1 characterized in that said first and second NAD(P) cofactor-linked oxidoreductase enzymes are the same enzyme.

3. The process of claim 1 characterized in that said second NAD(P) cofactor-linked oxidoreductase enzyme is alcohol dehydrogenase and said oxidase enzyme is alcohol oxidase or said second NAD(P) cofactor-linked oxidoreductase enzyme is horse liver alcohol dehydrogenase and said oxidase enzyme is alcohol oxidase.

4. The process of claim 1 characterized in that said second NAD(P) cofactor-linked oxidoreductase enzyme is glycerol dehydrogenase and said oxidase enzyme is glycerol oxidase or said second NAD(P) cofactor-linked oxidoreductase enzyme is sulfate reductase and said oxidase enzyme is sulfite oxidase.

5. The process of claim 1 characterized in that said second NAD(P) cofactor-linked oxidoreductase enzyme is malate dehydrogenase and said oxidase enzyme is malate oxidase or said second NAD(P) cofactor-linked oxidoreductase enzyme is aldehyde dehydrogenase and said oxidase enzyme is aldehyde oxidase.

6. The process of claim 1 characterized in that said second NAD(P) cofactor-linked oxidoreductase enzyme is glucose dehydrogenase and said oxidase enzyme is glucose oxidase or said second NAD(P) cofactor-linked oxidoreductase enzyme is xanthine dehydrogenase and said oxidase enzyme is xanthine oxidase.

7. The process of claim 1 characterized in that said second NAD(P) cofactor-linked oxidoreductase enzyme is lactate dehydrogenase and said oxidase enzyme is lactate oxidase or said second NAD(P) cofactor-linked oxidoreductase enzyme is oxalate dehydrogenase and said oxidase enzyme is oxalate oxidase.

8. The process of claim 1 characterized in that said second substrate is an aldehyde and said second reaction product is an alcohol or said second substrate is acetaldehyde and said second reaction product is ethanol.

9. The process of claim 1 characterized in that said second substrate is a ketone and said second reaction product is an alcohol or said second substrate is cyclohexanone and said second reaction product is cyclohexanol.

10. The process of claim 1 characterized in that said first substrate is cyclohexanol, said first and second NAD(P) cofactor-linked oxidoreductase enzyme is alcohol dehydrogenase, and said oxidase enzyme is alcohol oxidase.

**Patentansprüche**

1. Verfahren zur enzymatischen Oxidation eines ersten Substrates zur Bildung eines ersten Reaktionsproduktes welches umfaßt:

(1) Umsetzung eines ersten Substrates mit einer ersten NAD(P)-abhängigen Oxidoreduktase und NAD(P) zur Bildung eines ersten Reaktionsproduktes und NAD(P)$H_2$; wobei das erste Substrat durch die erste NAD(P)-abhängige Oxidoreduktase oxidiert wird, und

(2) anschließende Umsetzung eines zweiten Substrates und des in Stufe (1) gebildeten NAD(P)$H_2$ mit einer zweiten NAD(P)-abhängigen Oxidoreduktase zur Bildung eines zweiten Reaktionsproduktes und NAD(P); wobei das zweite Substrat durch die zweite NAD(P)-abhängige Oxidoreduktase reduziert wird, gekennzeichnet durch

(3) Umsetzung des zweiten Reaktionsproduktes mit Sauerstoff katalysiert durch eine Oxidase, um dadurch das zweite Substrat zu regenerieren.

2. Verfahren nach Anspruch 1, dadurch gekennzeichnet, daß die erste und zweite NAD(P)-abhängige Oxidoreduktase das gleiche Enzym sind.

3. Verfahren nach Anspruch 1, dadurch gekennzeichnet, daß die zweite NAD(P)-abhängige Oxidoreduktase Alkohol-Dehydrogenase und die Oxidase Alkohol-Oxidase ist, oder daß die zweite NAD(P)-abhängige Oxidoreduktase Pferdeleber-Alkohol-Dehydrogenase und die Oxidase Alkohol-Oxidase ist.

4. Verfahren nach Anspruch 1, dadurch gekennzeichnet, daß die zweite NAD(P)-abhängige Oxidoreduktase Glycerol-Dehydrogenase und die Oxidase Glycerol-Oxidase ist, oder daß die zweite NAD(P)-abhängige Oxidoreduktase Sulfat-Reduktase und die Oxidase Sulfit-Oxidase ist.

5. Verfahren nach Anspruch 1, dadurch gekennzeichnet, daß die zweite NAD(P)-abhängige Oxidoreduktase Malat-Dehydrogenase und die Oxidase Malat-Oxidase ist, oder daß die zweite NAD(P)-abhängige Oxidoreduktase Aldehyd-Dehydrogenase und die Oxidase Aldehyd-Oxidase ist.

6. Verfahren nach Anspruch 1, dadurch gekennzeichnet, daß die zweite NAD(P)-abhängige Oxidoreduktase Glucose-Dehydrogenase und die Oxidase Glucose-Oxidase ist, oder daß die zweite NAD(P)-abhängige Oxidoreduktase Xanthin-Dehydrogenase und die Oxidase Xanthin-Oxidase ist.

7. Verfahren nach Anspruch 1, dadurch gekennzeichnet, daß die zweite NAD(P)-abhängige Oxidoreduktase Lactat-Dehydrogenase und die Oxidase Lactat-Oxidase ist, oder daß die zweite NAD(P)-abhängige Oxidoreduktase Oxalat-Dehydrogenase und die Oxidase Oxalat-Oxidase ist.

8. Verfahren nach Anspruch 1, dadurch gekennzeichnet, daß das zweite Substrat ein Aldehyd und das zweite Reaktionsprodukt ein Alkohol ist, oder daß das zweite Substrat Acetaldehyd und das zweite

Reaktionprodukt Ethanol ist.

9. Verfahren nach Anspruch 1, dadurch gekennzeichnet, daß das zweite Substrat ein Keton und das zweite Reaktionsprodukt ein Alkohol ist, oder daß das zweite Substrat Cyclohexanon und das zweite Reaktionsprodukt Cyclohexanol ist.

10. Verfahren nach Anspruch 1, dadurch gekennzeichnet, daß das erste Substrat Cyclohexanol und die erste und zweite NAD(P)-abhängige Oxidoreduktase Alkohol-Dehydrogenase und die Oxidase Alkohol-Oxidase ist.

**Revendications**

1. Procédé pour l'oxydation enzymatique d'un premier substrat pour former un premier produit de réaction qui comprend:

(1) la mise en réaction d'un premier substrat avec une première enzyme oxydoréductase liée au cofacteur NAD(P) et de NAD(P) pour former un premier produit de réaction et $NAD(P)H_2$; le premier substrat étant oxydé par la première enzyme oxydoréductase liée au cofacteur NAD(P) et

(2) la mise en réaction ultérieure d'un second substrat et du $NAD(P)H_2$ produit dans l'étape (1) avec une seconde enzyme oxydoréductase liée au cofacteur NAD(P) pour former un second produit de réaction et NAD(P); le second substrat étant réduit par la seconde enzyme oxydo-réductase liée au cofacteur NAD(P), caractérisé par

(3) la mise en réaction du second produit de réaction avec de l'oxygène catalysé par une enzyme oxydase, pour régénérer ainsi le second substrat.

2. Procédé selon la revendication 1, caractérisé en ce que la première et la seconde enzymes oxydoréductases liées au cofacteur NAD(P) sont la même enzyme.

3. Procédé selon la revendication 1, caractérisé en ce que la seconde enzyme oxydoréductase liée au cofacteur NAD(P) est l'alcool-déshydrogénase et en ce que l'enzyme oxydase est l'alcool-oxydase ou en ce que la seconde enzyme oxydoréductase liée au cofacteur NAD(P) est l'alcool-déshydrogénase de foie de cheval et l'enzyme oxydase est l'alcool-oxydase.

4. Procédé selon la revendication 1, caractérisé en ce que la seconde enzyme oxydoréductase liée au cofacteur NAD(P) est la glycérol-déshydrogénase et l'enzyme oxydase est la glycérol-oxydase ou en ce que la seconde enzyme oxydoréductase liée au cofacteur NAD(P) est la sulfate-réductase et l'enzyme oxydase est la sulfite-oxydase.

5. Procédé selon la revendication 1, caractérisé en ce que la seconde enzyme oxydoréductase liée au cofacteur NAD(P) est la malate-déshydrogénase et l'enzyme oxydase est la malate-oxydase ou en ce que la seconde enzyme oxydoréductase liée au cofacteur NAD(P) est l'aldéhyde-déshydrogénase et l'enzyme oxydase est l'aldéhyde-oxydase.

6. Procédé selon la revendication 1, caractérisé en ce que la seconde enzyme oxydoréductase liée au cofacteur NAD(P) est la glucose-déshydrogénase et l'enzyme oxydase est la glucose-oxydase ou en ce que la seconde enzyme oxydoréductase liée au cofacteur NAD(P) est la xanthine-déshydrogénase et l'enzyme oxydase est la xanthine-oxydase.

7. Procédé selon la revendication 1, caractérisé en ce que la seconde enzyme oxydoréductase liée au cofacteur NAD(P) est la lactate-déshydrogénase et l'enzyme oxydase est la lactate-oxydase ou en ce que la seconde enzyme oxydoréductase liée au cofacteur NAD(P) est l'oxalate-déshydrogénase et l'enzyme oxydase est l'oxalate-oxydase.

8. Procédé selon la revendication 1, caractérisé en ce que le second substrat est un aldéhyde et le second produit de réaction est un alcool ou en ce que le second substrat est l'acétaldéhyde et le second produit de réaction est l'éthanol.

9. Procédé selon la revendication 1, caractérisé en ce que le second substrat est une cétone et le second produit de réaction est un alcool ou en ce que le second substrat est la cyclohexanone et le second produit de réaction est le cyclohexanol.

10. Procédé selon la revendication 1, caractérisé en ce que le premier substrat est le cyclohexanol, la première et la seconde enzymes oxydoréductases liées au cofacteur NAD(P) sont l'alcool-déshydrogénase et l'enzyme oxydase est l'alcool-oxydase.